# EUROPÄISCHE PATENTANMELDUNG

(11) **EP 0 888 771 A1**
(43) Veröffentlichungstag der Anmeldung: **07.01.1999**
(21) Anmeldenummer: 97110924.4
(22) Anmeldetag: 02.07.1997
(51) Int. Cl.: A61K 31/16, A61K 47/10, A61K 47/14

(54) **Zusammensetzung mit lokalanästhetischer Wirkung**

(71) Anmelder: Kress, Robert Christopher, 24768 Rendsburg (DE)
(72) Erfinder: Kress, Robert Christopher, 24768 Rendsburg (DE)
(74) Vertreter: Thiel, Christian, Dr. Dipl.-Chem.

(57) **Zusammenfassung**

Die Erfindung betrifft eine Zusammensetzung mit lokalanästhetischer Wirkung mit 85 bis 95 Gew.-% eines Trägeröls auf Fettsäurebasis, 2,5 bis 10 Gew.-% eines körperverträglichen Alkohols und 1,0 bis 10 Gew.-% Lidocain.

## Beschreibung

Die Erfindung betrifft eine Zusammensetzung mit lokalanästhetischer Wirkung, die insbesondere zur Verwendung als Posing-Oil im Bodybuilding-Sport verwendbar ist.

Es ist bekannt, im Bodybuilding-Sport den Körper mit einem sogenannten Posing-Oil einzureiben, um die Muskulatur herauszustellen und zu betonen. Dies wird insbesondere dadurch erreicht, daß das Öl einerseits einen matten Glanz verleiht, andererseits aber über Brechungs- und Reflexionserscheinungen Blickpunkte setzt, die den Muskelaufbau und das Muskeispiel betonen.

Als Posing-Oil werden herkömmlicherweise natürliche oder synthetische Fette und Öle eingesetzt, wie sie vor allem in der Kosmetik verwandt werden. Hinzu kommen auch spezielle Abmischungen, die häufig im Belieben des Anwenders liegen. Es ist bekannt geworden, daß eine Reihe dieser Öle in ihrer Zusammensetzung den normalen kosmetischen oder medizinischen Anforderungen (DAB; Deutsches Arzneimittelbuch) nicht genügen und zu Irritationen der Haut, Allergien oder sogar leichten Vergiftungserscheinungen führen können, sei es durch transdermale Effekte oder aber durch Einatmung der Dämpfe.

Hinzu können Probleme kommen, die sich aus der Unvereinbarkeit des Öls mit der Haut ergeben, dergestalt, daß die Hautatmung blockiert wird oder daß das Öl schweißunverträglich ist. Ersteres hat nachteilige physiologische Auswirkungen, letzteres ist nachteilig für die optischen Effekte.

Bei Verwendung allergener oder hautunverträglicher Öle kommt es zu Irritationen der Haut, die sich nachteilig auf die Performance auswirken.

Es ergeben sich ferner Situationen, bei denen im Wettbewerb durch Umwelteinflüsse, beispielsweise Zugluft, Irritationen der Haut oder Muskelzuckungen auftreten können. Solchen Einflüssen, die wettbewerbsverzerrend wirken können, kann nicht immer begegnet werden, auch können solche Einflüsse nicht immer zuverlässig ausgeschaltet werden.

Ziel der Erfindung ist deshalb die Bereitstellung einer Zusammensetzung, die als Posing-Oil verwandt werden kann, gut körperverträglich, schweißverträglich und atmungsaktiv ist, keine Allergien und sonstige toxische Einflüsse auslöst, eine lockernde Wirkung hat, Irritationen der Haut und Muskulatur auszuschließen vermag und darüberhinaus die für ein Körperöl verlangte kosmetische Wirkung aufweist.

Diese Ziel wird mit einer Zusammensetzung mit lokalanästhetischer Wirkung erreicht, das die folgenden Bestandteile aufweist:
85 bis 95 Gew.-% eines Trägeröls auf Fettsäurebasis,
2,5 bis 10 Gew.-% eines körperverträglichen Alkohols und
1,0 bis 10 Gew.-% Lidocain.

Es hat sich gezeigt, daß die erfindungsgemäße Zusammensetzung ideal als Posing-Oil für den Bodybuilding-Sport geeignet ist. Das Trägeröl auf Fettsäurebasis macht die Haut geschmeidig und bringt die erwünschten optischen Effekte mit sich. Gleichzeitig ist das Trägeröl ein ideales Trägermittel für die übrigen Bestandteile. Die Hautatmung wird nicht eingeschränkt. Das Trägeröl ist schweißverträglich, d. h. es wird durch Schweiß nicht weggewaschen oder abgetragen und bildet mit Schweiß auch keine Schlieren.

Der körperverträgliche Alkohol dient in erster Linie als Lösungsvermittler für den dritten Bestandteil. Gleichzeitig trägt er aber zur kosmetischen Wirkung bei.

Lidocain ist ein stark wirkendes Lokalanästhetikum, das über den Alkohol in der Zusammensetzung verteilt wird und die Haut unempfindlich gegenüber äußeren Einflüssen und Irritationen macht.

Zweckmäßigerweise handelt es sich bei dem Trägeröl um ein Triglycerid, insbesondere um ein solches auf Basis mittelkettiger, vorzugsweise gesättigter pflanzlicher Fettsäuren. Ein bevorzugtes Produkt wird von der Fa. Hüls unter der Bezeichnung 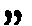 Miglyol^{R} 812" vertrieben.

Als körperverträglicher Alkohol kommt insbesondere Benzylalkohol in Frage, der nicht nur körperverträglich ist, sondern auch ein ausgezeichneter Lösungsvermittler. Darüberhinaus hat Benzylalkohol desinfizierende und schwache anästhetisierendeWirkung, ergänzt also das Lidocain in seiner Wirkung.

Zweckmäßigerweise werden alle Bestandteile in DAB-Qualität verwandt.

Besonders bevorzugt ist eine Zusammensetzung, die etwa 90 Gew.-% Miglyol 812, etwa 5 Gew.-% Benzylalkohol und etwa 5 Gew.-% Lidocainhydrochlorid, jeweils in DAB-Qualität, enthält.

Die erfindungsgemäße Zusammensetzung wird wie folgt hergestellt.

Zunächst wird das Trägeröl, vorzugsweise Miglyol 812, ein Triglycerid von C₈-C₁₂-Fettsäuren, auf 95°C erhitzt und unter Rühren 15 Minuten konstant auf dieser Temperatur gehalten. Anschließend wird zunächst der Benzylalkohol eingerührt und danach das Lidocainhydrochlorid. Lidocainhydrochlorid hat einen Schmelzpunkt von 78,9°C und löst sich bei der Zugabe in der heißen Mischung ohne weiteres auf.

Zur Homogenisierung und gleichmäßigen Verteilung wird die Zusammensetzung weitere 15 Minuten bei 95°C gehalten und gerührt. Anschließend wird die Zusammensetzung bei 95°C steril abgefüllt und luftdicht verschlossen. Nach dem Abkühlen ist die Zusammensetzung anwendungsfertig.

Es ist sinnvoll, die Abfüllung portionsweise vorzunehmen, so daß anwendungsfertige Einzelportionen erhalten werden.

## Patentansprüche

1. Zusammensetzung mit lokalanästhetischer Wirkung, insbesondere zur Verwendung als Posing-Oil, kennzeichnet durch die folgenden Bestandteile:
85 bis 95 Gew.-% eines Trägeröls auf Fettsäurebasis,
2,5 bis 10 Gew.-% eines körperverträglichen Alkohols und
1,0 bis 10 Gew.-% Lidocain.

2. Zusammensetzung nach Anspruch 1, dadurch gekennzeichnet, daß das Trägeröl ein Triglycerid ist.

3. Zusammensetzung nach Anspruch 2, dadurch gekennzeichnet, daß das Trägeröl ein Ester mittelkettiger, vorzugsweise gesättigter pflanzlicher Fettsäuren ist.

4. Zusammensetzung nach einem der vorstehenden Ansprüche, dadurch gekennzeichnet, daß der körperverträgliche Alkohol Benzylalkohol ist.

5. Zusammensetzung mit lokalanesthetischer Wirkung, bestehend aus 90 Gew.-% Triglyceriden mittelkettiger pflanzlicher Fettsäuren, 5 Gew.-% Benzylalkohol und 5 % Lidocain.

6. Verwendung der Zusammensetzung eines der vorstehenden Ansprüche als Posing-Oil im Bodybuilding-Sport.
